# EUROPEAN PATENT APPLICATION

(11) **EP 3 771 473 A1**
(43) Date of publication of application: **03.02.2021**
(21) Application number: 19188894.0
(22) Date of filing: 29.07.2019
(51) Int. Cl.: A61M 1/10, A61M 1/12

(54) **CARDIAC ASSIST DEVICE**

(71) Applicant: ETH Zurich, 8092 Zurich (CH); Fondazione Cardiocentro Ticino (FCCT), 6900 Lugano (CH)
(72) Inventor: JENNY, Patrick, 8872 Weesen (CH); KUMMER, Thomas, 8154 Oberglatt (CH); DEMERTZIS, Stefanos, 6900 Lugano (CH); VANDENBERGHE, Stijn, 6900 Lugano (CH); CONRAD, Jonas, 8050 Zürich (CH); LIEM, Michael, 8052 Zürich (CH)

(57) **Abstract**

The present invention relates to a cardiac assist device for contracting a human or animal heart, comprising a first patch configured to be placed from the outside onto the heart, a second patch configured to be placed from the outside onto the heart, and a mechanical adjustment mechanism for mechanically adjusting the positional relationship between the first patch and the second patch so as to contract the heart situated therebetween.

## Description

### Technical Field

The present invention relates to a cardiac assist device for contracting a human or animal heart.

### Prior Art

In emergency units, during surgeries or in intensive care, the human heart may stop beating. If the thorax of the patient is open, the heart may be manually massaged by a doctor to maintain blood circulation through the patient's body. Such a manual heart massage not only requires high endurance of the doctor, but also demands the doctor's attention, keeping him/her away from addressing other crucial tasks.

WO 2004/093731 A2, WO 00/13722 and DE 10 2008 018 919 A1 relate to fluid actuated devices for assisting the human heart in its pumping function. Furthermore, WO 2009/048397 A1 relates to a compression device with a housing for accommodating a portion of a human heart, and an operating element for compressing the human heart in the housing.

### Summary of the invention

The present invention relates to a cardiac assist device for contracting a human or animal heart. The cardiac assist device may be configured to contract a human, other mammal, bird or other heart, which is devided in four chambers with upper left and right atria and lower left and right ventricles. The cardiac assist device may be an emergency device, which is intended and configured to be used when the thorax of the patient is open, e.g. in emergency situations during surgery. Alternatively or additionally, the cardiac assist device may be intended and configured to be used as a long-term heart assist device that the patient permanently carries for multiple days, weeks, months or years. In the latter case, the cardiac assist device may be configured to be used when the thorax of the patient is closed.

The cardiac assist device may be configured to take over the entire pumping function of the heart and/or to assist a heart with weakened pumping functionality. The cardiac assist device may be configured to maintain sufficient blood circulation through the patient's vasculature so as to ensure survival of the patient even if the heart stops functioning completely. The cardiac assist device may assist in and/or completely provide the systole of the ventricles of the heart. Additionally or alternatively, the cardiac assist device may assist in and/or completely provide the diastole of the ventricles of the heart.

The cardiac assist device comprises a first patch configured to be placed from the outside onto the heart, optionally onto the left ventricle of the heart, e.g. onto the epicardium of the left ventricle. Furthermore, the device comprises a second patch configured to be placed from the outside onto the heart, optinally onto the right ventricle of the heart, e.g. onto the epicardium of the right ventricle. The heart comprises a pericardium, which is a fibroserous sac covering the heart. Furthermore, the heart comprises the so-called myocardium, wherein the outer most layer of the myocoradium is the epicardium. The pericardium surrounds the epicardium with the pericardial fluid therebetween. When opening the thorax, the pericardium is seen, wherein the epicardium is hidden inside the pericardium. Access to the epicardium can be gainged by opening the pericardial sac. A patch according to the invention may be an element, which holistically exhibits a substantially plate-like shape, wherein the plate may be in its entirety or partially flat and/or bent. The patch may have an overall planar and/or plane like shape, wherein portions of the patch may be bent. The patch may be bendable to adjust its shape to the heart it is attached to. Alternatively, the patch may be rigid. The matter of fact that a patch is configured to be placed onto the left and/or right ventricle implies that it is specifically adapted, e.g. in its shape, material and/or functionality, to the respective ventricle of the heart so as to allow the cardiac assist device to fulfill its intended function. The patch may be formed from a single element. In an embodiment, the patch is not a housing.

The patches may exhibit extensions in the width and in the height directions, which are significantly larger than the extension of the patch in the depth/thickness direction. An extension of the patch in one or all of these directions may be determined via an orthographic projection of the patch on a plane, which is parallel to that specific direction. Optionally, the extensions in the depth/thickness direction is less than 50%, preferably less than 40%, even more preferably less than 35% smaller than the extension of the patches in the smaller one of the width and the height directions. In an embodiment, the patch encircles less than 50%, preferably less than 40% of the heart's circumference when the patch is attached to the heart in the intended fashion.

Furthermore, the cardiac assist device comprises a mechanical adjustment mechanism for mechanically adjusting the positional relationship between the first patch and the second patch so as to contract the heart situated therebetween. In addition, the mechanical adjustment mechanism may be configured to adjust the positional relationship between the first patch and the second patch to expand the heart situated therebetween. To adjust the positional relationship between the two patches, a distance and/or an orientation of the two patches with respect to each other may be adapted. In other words, a volume enclosed by the first and second patches may be adapted by the mechanical adjustment mechanism for compressing the heart in between those two patches. The mechanical adjustment implies that the positional relationship between the two patches is changed via mechanical means, preferably only via mechanical means. In an embodiment, the positional relationship is not changed via fluid dynamic, electric, electromagnetic and/or other non-mechanical means.

The cardiac assist device according to the present invention may assist a heart in its pumping function and/or overtake the pumping function completely. In emergency situations, the device according to the present invention may replace manual heart massages by a doctor, thereby allowing the doctor to focus on other important tasks. At the same time, the cardiac assist device according to the present invention may impose a stable contraction pattern on the heart, which is identical to the contraction pattern of a natural heart, which results in excellent treatment safety and reliability. Furthermore, the mechanical actuation of the adjustment mechanism avoids blood of the patient coming into contact with technical surfaces, thereby eliminating the risk of blood clutting. In addition, the patches advantegously induce a pulsatile blood flow. It also allows the device to be relatively small, therefore allowing for an easy implantation thereof in the narrow space around the heart. In addition, the cardiac assist device according to the present invention does not require pressurized air, currents and/or other potentially harmful substances in the proximity of the heart. Finally, the matter of fact that the cardiac assist device comprises patches for engaging with the heart results in an easy and quick mountability, at the same time allowing for an operation of the device with high accuracy.

According to an embodiment, the mechanical adjustment mechanism comprises an abutment element, which is mechanically connected to one of the first and second patches. The abutment element may be configured to absorb pressure forces exerted thereon, e.g. by the heart. In this embodiment, the cardiac assist device further comprises a pulling element, which is movable with respect to the abutment element, the pulling element being mechanically connected to the other one of the first and second patches. In the cardiac assist device according to this embodiment, the pulling element may be moved relatively to the abutment element to exert a pressure force onto the heart with the patch it is connected to, wherein the abutment element provides a counterforce via the other patch, resulting in the heart situated between the two patches to be contracted. The pulling element and the abutment element may be detachably or non-detachably connected to the first and second patches. This embodiment provides a mechanical adjustment mechanism with low complexity as well as high reliability.

According to an embodiment, the adjustment mechanism comprises a further pulling element mechanically connected to the other one of the first and second patches, i.e. the patch to which the other pulling element is connected to. The device according to this embodiment is configured such that the pulling element and the further pulling element may run from one of the first and second patches to the other one of the first and second patches on opposite sides of the heart, e.g. one pulling element on the posterior side and one pulling element on the anterior side of the heart. The cardiac assist device may also comprise a further abutment element, which may be connected to the patch the other abutment element is connected to. One of the abutment elements may be provided to mainly provide a counterforce for one of the pulling elements, and the further abutment element may be provided to mainly provide a counterforce for the further pulling element. This embodiment allows for a particularly symmetric and uniform contraction of the heart. However, if an asymmetric contraction is aspired, i.e. that one pulling element moves less than the other, the embodiment allows for this as well.

According to an embodiment, the cardiac assist device comprises a quick lock for mechanically connecting and detaching one of the pulling elements from one of the first and second patches. A quick lock may be a lock, which may be operated by an operator without the assistance of any tools. In other words, the quick lock may be hand-operated. Providing one of the pulling elements in a detachable fashion from one of the patches via a quick lock allows for a simple and quick attachment of the cardiac assist device to the heart. This is particularly advantageous if the cardiac assist device is used in emergency situations, in which quick fixation of the assist device to the heart is critical. It is also conceivable that both pulling elements are connectable and detachable from one of the patches via a quick lock. The quick lock may be a magnetic lock. Additionally or alternatively, the quick lock may comprise a velcro fastener, a clamping fastener, a fastener in which two complementary elements are detachably engaged with each other via a form fit and/or any other type of fastener.

According to an embodiment, the abutment element of the adjustment mechanism is formed as a sleeve and the pulling element as a cable running inside the sleeve. The combination of abutment elements and pulling elements may therefore be provided by a Bowden cable. The sleeve may exhibit a large enough compressive strength so as to absorb pressure forces exerted thereon by the pulling member via the first and second patches and the heart. The cable may be formed from a metallic material, e.g. steel and/or titanium, glass fibre, carbon and/or plastic. The sleeve may comprise different layers, e.g. a support layer for providing mechanical stability, e.g. formed by steel wires, and a protective outer housing, e.g. formed by plastic and/or rubber. The sleeve and/or the cable may be bendable but not compressible in lengthwise direction. This embodiment results in a simple but reliable mechanical adjustment mechanism, which exhibits high flexibiltiy due to the bendabiltiy of the sleeve. However, it is also conceivable that the pulling element is formed as a rigid bar, which may be slidably supported in the abutment element, which may be formed as a rigid tube.

According to an embodiment, the first patch comprises a contact surface for contacting the heart, optionally the left ventricle of the heart, and the second patch comprises a contact surface for contacting the heart, optionally the right ventricle of the heart. The contact surfaces may be those portions of the respective patches, which engage with the heart, optionally the left and right ventricles of the heart, during the intended use of the cardiac assist device. According to this embodiment, the contact surface of the first patch, e.g. the contact surface for engaging with the left ventricle, is smaller in size than the contact surface of the second patch, e.g. the contact surface for engaging with the right ventricle. This configuration ensures that e.g. the pressure exerted on the more vulnerable right ventricle is reduced, at the same time providing a large enough pressure for effective contraction to the stronger left ventricle. The relatively large contact surface at the right ventricle may e.g. further ensure that the right ventricle substantially sustains its shape during application of the device. The contact surfaces may be respectively formed three-dimensionally so as to ideally conform to the left and right ventricle, respectively.

According to an embodiment, the cardiac assist device further comprises a fixation device for fixing, e.g. attaching, at least one of the patches to the heart. According to an embodiment, each of the patches comprises a fixation device for fixing the respective patch to the heart. The fixation device according to this embodiment may be configured to fix the patch in position relative to the heart despite of the impact of gravity and loading exerted on the patch during the intended use of the cardiac assist device. This embodiment ensures a reliable operation of the cardiac assist device, as the position and/or orientation of the device relative to the heart is ensured throughout operation. Furthermore, this embodiment may ensure that the two patches are not displaced relatively to the heart in an undesired fashion, if the cardiac assist device is turned off or undergoes failure.

According to an embodiment, the fixation device comprises a suction port provided at the patch for fixing the patch to the heart via vacuum. The suction port may be provided on the contact surface of the patch, which is in contact with the heart, e.g. the epicardium of the heart. The suction port may be provided at the centre of the contact surface, for example. It is also conceivable that the patch comprises multiple suction ports, which may be situated at arbitrary positions on the contact surface. The patch may also comprise a vacuum input port, which is fluidly connected to the suction port. The vacuum input port may be easily accessable if the patch is attached to the human heart in the intended fashion. The vacuum input port may be provided at a side surface of the patch and/or on a surface opposite the contact surface, for example. Fixing the patch via vacuum to the heart provides a reliable and non-invasive way of fixing the positional relationship between the patch and the heart. In particular, with this fixation method, the heart wall, e.g. the epicardium, is not penetrated while a reliable fixation of the patch e.g. on the slippery epicardium is provided.

According to an embodiment, the cardiac assist device comprises a vacuum source, which is fluidly connected to the suction port, e.g. via the vacuum input port, for generating a vacuum, e.g. with a pressure of 20 % below atmospheric pressure. The vacuum source may be fluidly connected to suction ports of both patches to provide a vacuum for the first and second patches with a single vacuum source. The vacuum may be created by an ejector, which may be pneumatically driven, by a pump and/or differently. The vacuum source may be provided remote from the patient, in particular outside of the patient's torax.

According to an embodiment, the fixation device comprises a needle for piercing through the heart wall and an unfoldable retaining element at the needle's tip. The retaining element may be retracted when piercing the needle through the heart wall, and unfolded once the tip of the needle has passed through the entire wall to provide effective retention. In an embodiment, the retaining element may be unfolded in a space behind the heart wall inside of the heart. The retaining element may be an umbrella-like structure and/or an inflatable balloon and/or an arbitrary other structure providing the above-described retaining effect. According to an embodiment, the retaining element may be retracted to allow for a safe removal of the patch from the heart once the use of the cardiac assist device is completed. The embodiment provides the advantage that pressurized air systems are not required for use of the cardiac assist device. The configuration of the fixation device according to this embodiment may therefore be well-suited for long term ventricular heart assist devices.

According to an embodiment, the adjustment mechanism comprises an actuation device connected to the pulling elements and/or the pulling elements, and a control unit for controlling the actuation device. The actuation device may be a linear motor, a linear actuator and/or a servo-motor, which may be driven by an electric motor. The actuation device may be provided remote from the patient, i.e. outside of the patient's thorax. The patches may be connected to the linear actuator via the pulling element(s).

Configuring the actuation device via a linear actuator, the adjustment mechanism may be distance/displacement controlled, thereby allowing for the control of a contraction pattern of the patches with high accuracy. According to an embodiment, the control unit is configured to vary the positional relationship, e.g. the distance, between the first and second patches via the actuation device periodically. According to an embodiment, the control unit is configured to vary the positional relationship along a sinusoidal function, which may be a function that is definable by x(t) = Asin (Ωt + ϕ) + C, e.g. along a sin² function. The function may be configured to lead to 40-150, preferably 60-100, more preferably 70-80, even more preferably 72-75 contractions/beats per minute of the heart. Furthermore, the angular frequency Ω can be chosen differently for the contraction and the relaxation phases of the heart, allowing the device to contract faster and relax slower such that the heart has enough time for refilling.

According to an embodiment, the control unit of the cardiac assist device is configured to adjust the control of the actuation device based on a measured heart performance factor. In this regard, it is conceivable that a sensing unit for sensing the heart performance factor is attached to the heart and electrically connected to the control device. The control device may compare the measured heart performance factor with an aspired heart performance factor, e.g. stored in a memory of the control unit, to adjust the control of the actuation device based on the comparison. The heart performance factor may be a pressure-volume loop, i.e. a p-V loop, or a parameter/combination of parameters from which a p-V loop of the heart is derivable. Other heart performance factors are also conceivable in this regard. According to this embodiment, an optimal performance of the cardiac assist device may be ensured by adjusting the operation of the device to achieve optimal blood circulation performance in the patient's body.

According to an embodiment, the control unit is configured to synchronize the adjustment of the positional relationship between the first and second patches with a measured contraction frequency of the heart. In this embodiment, a sensing device for sensing the natural contraction frequency of the heart may be attched to the heart and electrically connected to the control unit. The control unit may control the actuation device and therefore the positional relationship between the first and second patches based on the measured natural contraction frequency. For example, an electrocardiograph machine can be coupled with the control unit. Additionally or alternatively, a ventricular pressure value could be measured by injecting a needle into at least one heart chamber. Thus, a given temporal pressure evolution inside the heart may be measured. The control unit may be configured to match the measured pressure evolution by respectively controlling the positional relationship between the first and second patches. This embodiment ensures that the cardiac assist device allows for an optimal synchronisation between a natural pumping of the heart, which may still be present but weakend, and an assistive pumping of the cardiac assist device attached to the heart. Optimal performance of the cardiac assist device is thus ensured.

Furthermore, the invention relates to a method of attaching a cardiac assist device according to any of the above embodiments to a human or animal heart, comprising the step of moving one of the first and second patches around the heart, optionally along the heart's posterior side, wherein the patches may be coupled with each other by a first coupling means, e.g. a first Bowden cable, at said posterior side, and, optionally, coupling the first and second patches with a second coupling means, e.g. a second Bowden cable, at the anterior side of the heart. The method may further comprise a step of attaching the first patch to the heart, optionally to the left ventricle, e.g. to the epicardium of the left ventricle, and a step of attaching the second patch to the heart, optionally to the right ventricle, e.g. to the epicardium of the right ventricle. The attaching of one of the first and second patches to heart, e.g. to the epicardium of the heart, may be conducted prior to moving the other one of the first and second patches around the heart. In addition, the invention relates to a method of using a cardiac assist device according to any of the above embodiments as a long-term cardiac assist device. The invention also relates to a method of using the cardiac assist device according to any of the above embodiments as an emergency cardiac assist device, e.g. during surgery and/or in intensive care. Furthermore, the invention relates to a method of massaging a human or animal heart, comprising a step of manually massaging the heart, at least one of the above described steps of attaching the cardiac assist device to the heart, and a step of massaging the heart with the attached cardiac assist device, preferably in this order.

### Brief description of figures

Fig. 1 schematically shows a cardiac assist device according to an embodiment of the present invention.
Fig. 2 schematically shows a patch assembly of the cardiac assist device of Fig. 1 attached to a human heart in a perspective side view.
Fig. 3 schematically shows the patch assembly of the cardiac assist device of Fig. 2 attached to the human heart in a further perspective side view.
Fig. 4 schematically shows the patch assembly of the cardiac assist device of Fig. 2 attached to the human heart in a bottom view.
Fig. 5 schematically shows a fixation device for fixing the patches of the cardiac assist device of Fig. 1 to the human heart according to an embodiment.
Fig. 6 schematically shows a fixation device for fixing the patches of the cardiac assist device of Fig. 1 to the human heart according to a further embodiment.
Fig. 7 schematically shows an actuation assembly of the cardiac assist device of Fig. 1 in a top view with removed lid.

### Detailed description of embodiments

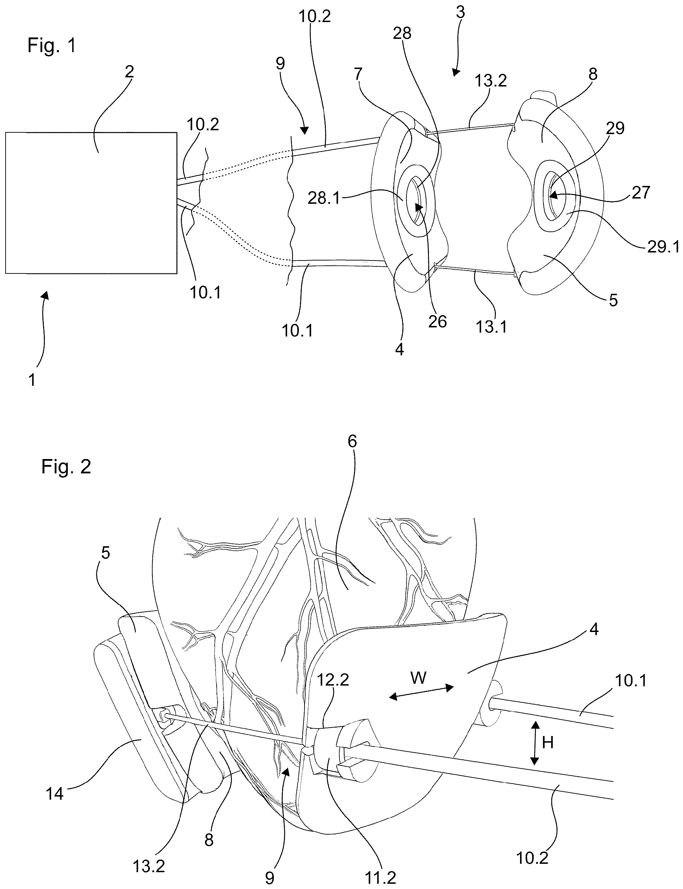

Fig. 1 shows a cardiac assist device 1 according to an embodiment of the present invention. The cardiac assist device 1 comprises an actuation assembly 2 and a patch assembly 3, both of which will be described in detail in the following. The cardiac assist device 1 is configured such that the patch assembly 3 may be positioned inside a patient's body, i.e. inside a patient's thorax, and such that the actuation assembly 2 may be situated outside of the patient's body. The actuation assembly 3 and the patch assembly 2 are connected with each other via an adjumstment mechanism 9, which will be described in detail in the following.

As derivable from Figs. 2 to 4, the patch assembly 3 comprises a first patch 4, which is configured to be positioned from the outside onto the epicardium of the left ventricle of the heart 6. In addition, the patch assembly 3 comprises a second patch 5, which is configured to be placed from the outside onto the epicardium of the right ventricle of the heart 6. In the present embodiment, the patches 4, 5 are manufactured by additive manufacturing, e.g. 3D printing. The patches 4, 5 may be made from plastic, e.g. polylactide (PLA), titanium and/or different materials. Multiple pairs of first and second patches 4, 5 may be provided, each pair being adapted for a specific heart size. A first set of first and second patches may be configured for a rather small heart, a second set of patches may be configured for a heart of average size, and a third set of first and second patches may be configured for a rather large heart.

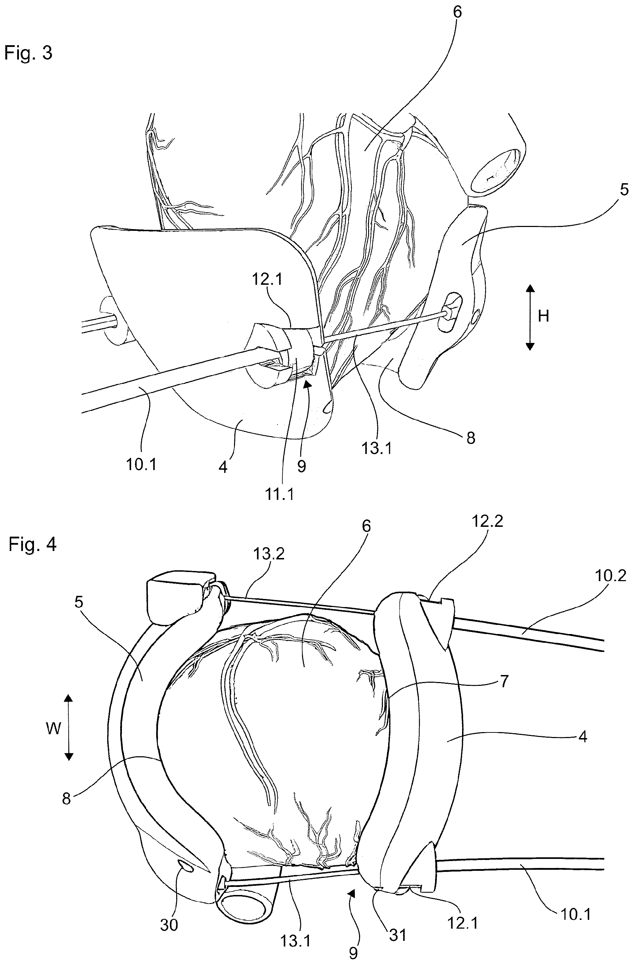

The first patch 4 may have a width W, which is larger than the width of the heart 6, and a height H, which approximately corresponds to the height of the left ventricle of the heart 6. In addition, the second patch 5 may have a width, which is larger than the width of the heart 6, and a height, which approxiametely corresponds to the height of the right ventricle of the heart 6. The width of the patches 4, 5 may be between 5-10 cm, preferably between 7-8 cm, and the height of the patches 4, 5 may be between 4-8 cm, preferably approximately 6.5 cm. Both patches 4, 5 exhibit a contact surface 7, 8 for engaging with the epicardium of the left and right ventricles of the heart 6, respectively. As derivable from Figs. 1 to 4, the entire patches 4, 5 including the contact surfaces 7, 8 exhibit a curvature in the width direction W so as to conform to the natural shape of the left and right ventricles of the heart 6. The second patch 5 may exhibit a larger curvature in width direction W than the first patch 4, as derivable from Fig. 4, for example.

Furthermore, the cardiac assist device 1 comprises a mechanical adjustment mechanism 9, which connects the patch assembly 3 with the actuation assembly 2. The adjustment mechanism 9 is configured to adjust the positional relationship between the first and second patches 4, 5. The adjustment mechanism 9 comprises two Bowden cables, each comprising a sleeve 10.1, 10.2 and a cable 13.1, 13.2. The sleeves 10.1, 10.2 are supported by the first patch 4 at one end and by the actuation assembly 2 at the other end. The sleeves 10.1, 10.2 constitute abutment elements according to the present invention. Each one of the sleeves 10.1, 10.2 comprises a cap 11.1, 11.2 provided at one end thereof, the cap 11.1, 11.2 being clamped into a complementary depression 12.1, 12.2 formed in the first patch 4. In height direction H, the depressions 12.1 and 12.2 may be provided approximately in the middle of the first patch 4. In width direction W, the depressions 12.1, 12.2 are provided at the right and left ends of the first patch 4.

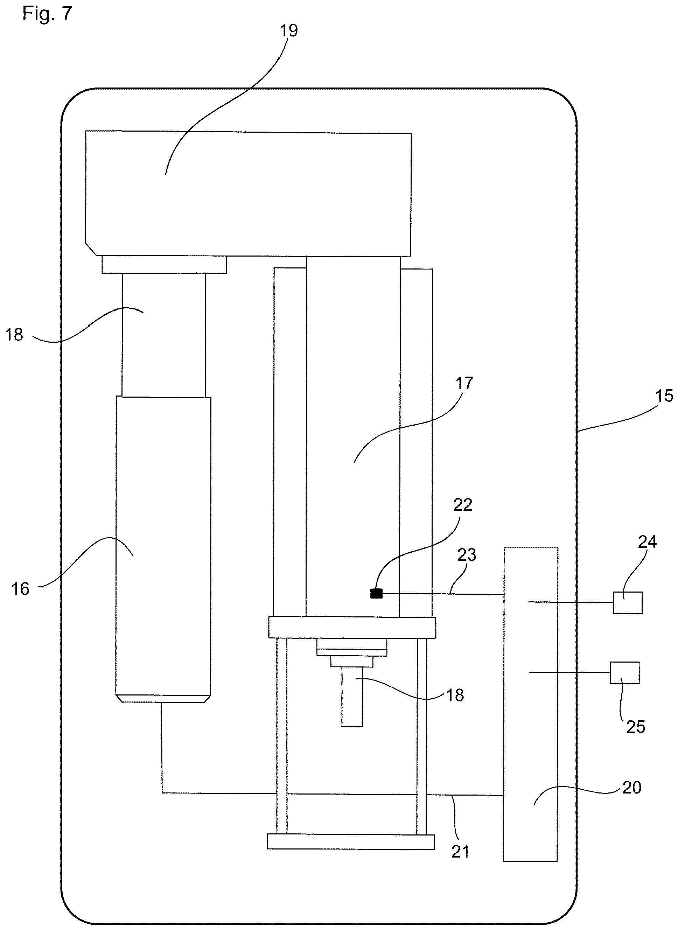

In addition, the Bowden cables of the mechanical adjustment mechanism 9 comprises two pulling members 13.1 and 13.2, which are formed as cables in the present embodiment. The cables 13.1 and 13.2 are slidably provided in the sleeves 10.1 and 10.2, respectively. The cables 13.1, 13.2 are attached to the second patch 5 at one end and to the acutation assembly 2 at the other end. In height direction H, the cables 13.1 and 13.2 may be connected to the second patch 5 approximately in the middle. In width direction W, the cables 13.1, 13.2 may be connected to end portions of the second patch. Specifically, the cables 13.1 and 13.2 may be connected to the second patch 5 in a movable manner to be rotatable around an axis of rotation, which is oriented approximately perpendicular to the height direction H, e.g. with an angle smaller 30°, when the the cardiac assist device 1 is attached to the human heart 6 in the intended fashion, as illustrated in Figs. 2 and 3. From the second patch 5, the cables 13.1 and 13.2 run through the sleeves 10.1 and 10.2 towards the actuation assembly 2, which is described in detail in the following in connection with Fig. 7. The cable may be formed from a metallic material, e.g. steel or titanium, glass fibre, carbon and/or plastic.

As derivable from Figs. 1 to 4, the cardiac assist device 1 is configured such that the cables 13.1 and 13.2 may run from the first 4 to the second patch 5 on opposite sides of the heart 6, namely on the anterior side (cable 13.2) and the posterior side (cable 13.1). The cardiac assist device 1 further comprises a quick lock 14 for connecting and detaching the cable 13.2 to the second patch 5. The quick lock 14 may be configured to fix the cable 13.2 to the second patch 5 magnetically, e.g. via a magnetic clasp. For mounting the cardiac assist device 1 to the human heart 6, the second patch 5 must only be moved around the posterior side of the heart 6, and then the quick lock 14 is actuated to connect the cable 13.2 to the second patch 5, the human heart 6 thereby be surrounded by the first and second patches 4, 5 on the left and right sides and the cables 13.1, 13.2 on the anterior and posterior sides. As derivable from Fig. 4, the patches 4, 5 are not in direct mechanical contact with each other, as they are provided remote from each other on opposite sides of the heart 6. The patches 4, 5 are merely coupled with each other via the Bowden cables 10.1, 10.2, 13.1, 13.2.

As derivable from Fig. 7, the actuation assembly 2 comprises a housing 15 comprising an electric motor 16 and a linear actuator 17, e.g. a linear spindle. The electric motor 16 is operatively connected with the linear actuator 17 via a gearbox 18 and a parallel unit 19. By operating the electric motor 16, a head 18 of the linear actuator 17 may be moved translatory. With the head 18 of the linear actuator 17, the cables 13.1 and 13.2 of the Bowden cables of the adjustment mechanism 9 are mechanically connected. Therefore, by operating the electric motor 16, the cables 13.1 and 13.2, which are coupled to the second patch 5, may be displaced inside the sleeves 10.1 and 10.2, which are coupled with the first patch 4, thereby adjusting the positional relationship between the first and second patches 4, 5. Specifically, by retracting the linear actuator 17, the second patch 5 is pulled via the cables 13.1, 13.2 towards the first patch 4, which stays approximately stationary, thereby reducing a space between the first and second patches 4, 5 to contract a heart situated therebetween. By extending the linear acutator 17, the second patch 5 is pushed away from the first patch 4 via the cables 13.1, 13.2, thereby increasing a space between the first and second patches 4, 5 to expand and/or allow a heart 6 to self-expand situated therebetween.

Furthermore, the housing 15 comprises a control unit 20, which is electrically connected with the electric motor 16 via a cable 21. The control device 20 may be configured to control the electric motor 16 such that the linear actuator 17 changes the positional relationship between the first and second patches 4, 5 in a periodic fashion, e.g. along a sin² profile. The linear actuator 17 may comprise a sensing unit 22, which is connected via a cable 23 to the control unit 20, for sensing a traveled distance/displacement of the linear actuator 17. The control unit 20 may be configured to control the linear actuator 17 via the electric motor 16 in a displacement feedback mode, thereby providing a displacement controlled linear actuator 17.

In addition, the control unit 20 may be connected with a sensing device 24 for determining a heart performance factor, e.g. a pressure-volume loop (measured p-V loop), of the heart to be treated, e.g. of the left ventricle of said heart. The control unit 20 may comprise a memory (not shown), in which an aspired performance factor, e.g. an aspired pressure-volume relationship (aspired p-V loop), is stored. The control unit 20 may be configured to compare the measured performance factor, e.g. the measured p-V loop, with the aspired performance factor, e.g. the aspired p-V loop, and adjust the control of the electric motor 16 and therefore the linear actuator 17 to move the measured performance factor towards the aspired performance factor, e.g. to align the measured with the aspired performance factor, for optimizing performance of the cardiac assist device 1.

Furthermore, the control unit 20 may be connected to a sensing device 25 for determining the natural pumping frequency of the heart 6 to be treated. The control device 20 may be configured to synchronize the frequency of change in the positional relationship between the first and second patches 4, 5 - by respectively controlling the electric motor 16 and thus the linear actuator 17 - with the measured pumping frequency of the heart 6 detected via the sensing device 25. Accordingly, the cardiac assist device 1 may be configured to optimally assist the heart 6 in its natural behavior.

In addition, the cardiac assist device 1 comprises a first fixation device 26 on the first patch and a second fixation device 27 on the second patch 5 for fixing the respective patch 4, 5 to the left and right ventricles of the heart 6, respectively. In the present embodiment, the fixation devices 26, 27 are provided at approximately the centres of the first and second patches 4, 5. The fixation devices 26, 27 of the present embodiment allow the cardiac assist device 1 to assist the heart 6 also during diastole. Specifically, when the distance between the first and second patches 4, 5 is increased, the fixation devices 26, 27 maintain a mechanical connection between the patches 4, 5 and the heart 6, thereby pulling on the heart and assisting in or even taking over of the expansion of the heart.

According to the embodiment shown in Figs. 1 to 4, the fixation devices 26, 27 comprise a suction port 28, 29 for fixing the patches 4, 5 to the heart 6 via vacuum. The suction ports 28, 29 respectively comprise a vacuum seal 28.1, 29.1, which may be formed of a flexible material, e.g. rubber, for engaging with the epicardium of the left and right ventricles, respectively. The suction ports 28 and 29 are respectively in fluid connection with a vacuum inlet port 30 and 31, which are illustrated in Fig. 4, for example. The vacuum inlet ports 30 and 31 may be Luer ports. The vacuum inlet ports 30, 31 are situated on a side surface of the patches 4, 5, namely on the side surface opposite to the quick lock 14. The vacuum ports 30, 31 may be connected via tubes to a vacuum source (not shown), which may be provided in the housing 15 of the actuation assembly 2. The vacuum source may be configured as an ejector and/or as a vacuum pump, which may be pneumatically driven, e.g. by pressurized air as it is usually available in medical facilities. The suction ports 28, 29 may occupy approximately half of the height and/or a third of the width of the patches 4, 5. Between the patches and the ejector/pump, a filter may be provided in order to protect the pump/ejector from heart liquids sucked in.

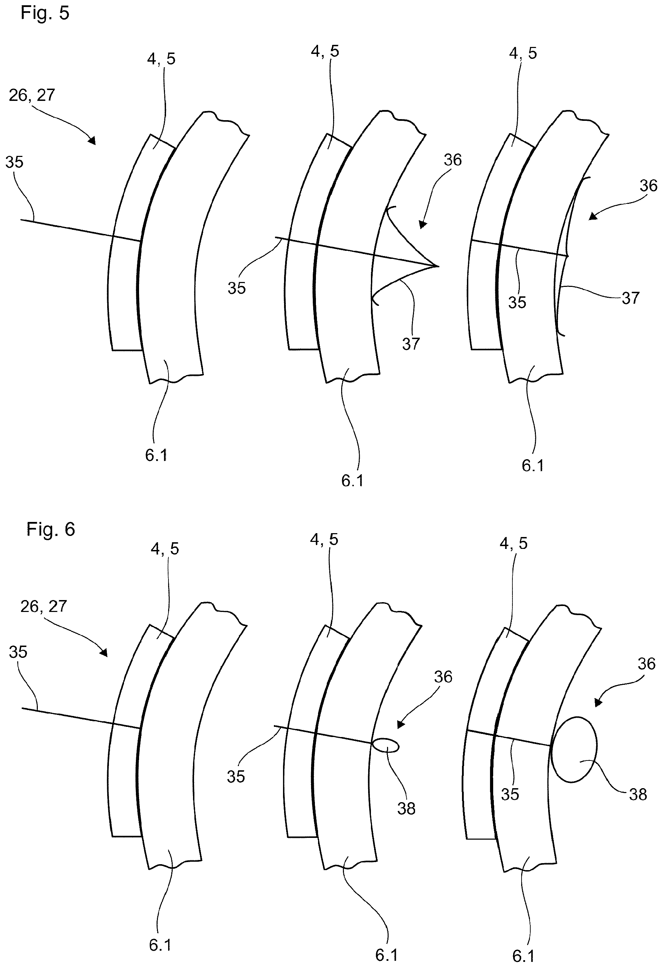

Alternatively, the fixation devices 26, 27 of the first and second patches 4, 5 may be configured as shown in Figs. 5 and/or 6. Specifically, the fixation devices 26, 27 may comprise a needle 35 and a retaining element 36. The needle 35 may be mechanically connected with the patch 4, 5 and may be pierced through the wall of the heart 6.1. While piercing the needle 35 through the wall of the heart 6.1, the retaining element 36 may be retracted. Once the needle 35 has been pierced through the wall of the heart 6.1 completely, the retaining element 36 may be unfolded to fix the patch 4, 5 in position. In the embodiment shown in Fig. 5, the retaining element 36 may be an umbrella-like structure 37, which may be unfolded by pulling the needle 35 backwards, once it has been pierced completely through the wall of the heart 6.1. In the embodiment shown in Fig. 6, the retaining element 36 may be a balloon 38, which may be inflated, once the needle 35 has been pierced completely through the wall of the heart 6.1. In the embodiment shown in Fig. 5, the umbrella-like structure 37 can be retracted again to remove the needle 35 from the wall 6.1 of the heart 6 and therefore release the patch 4, 5 from the heart 6. Likewise, in the embodiment shown in Fig. 6, the balloon 38 can be deflated to pull the needle 35 out of the wall 6.1 of the heart 6.

### Reference signs

- 1: Cardiac assist device
- 2: Actuation assembly
- 3: Patch assembly
- 4, 5: First, second patch
- 6. 6.1: Heart, heart wall
- 7, 8: First, second contact surface
- 9: Mechanical adjustment mechanism
- 10.1, 10.2: Sleeve
- 11.1, 11.: Sleeve cap
- 12.1, 12.2: Patch depression
- 13.1, 13.2: Pulling cable
- 14: Quick lock
- 15: Housing
- 16: Electric motor
- 17: Linear actuator
- 18: Gearbox
- 19: Parallel unit
- 20: Control unit
- 21, 23: Cable
- 24, 25: Sensing unit
- 26, 27: First, second fixation device
- 28, 29: Vacuum seal
- 30, 31: Vacuum inlet port
- 35: Needle
- 36: Retaining element
- 37: Umbrella-like structure
- 38: Balloon

## Claims

1. Cardiac assist device (1) for contracting a human or animal heart (6), comprising
a first patch (4) configured to be placed from the outside onto the heart (6), optionally onto the left ventricle of the heart (6);
a second patch (5) configured to be placed from the outside onto the heart (6), optionally onto the the right ventricle of the heart (6); and
a mechanical adjustment mechanism (9) for mechanically adjusting the positional relationship between the first patch (4) and the second patch (5) so as to contract the heart (6) situated therebetween.

2. The cardiac assist device (1) according to Claim 1, wherein the mechanical adjustment mechanism (9) comprises an abutment element (10.1), which is connected to one of the first and second patches (4), and a pulling element (13.1) movable with respect to the abutment element (10.1), which is connected to the other one of the first and second patches (5).

3. The cardiac assist device (1) according to Claim 2, the mechanical adjustment mechanism (9) comprising a further pulling element (13.2) connected to the other one of the first and second patches (5), wherein the device (1) is configured such that the pulling element (13.1) and the further pulling element (13.2) may run from one of the first and second patches (4) to the other one of the first and second patches (5) on opposite sides of the heart (6).

4. The cardiac assist device (1) according to Claim 3, further comprising a quick lock (14) for connecting and detaching one of the pulling elements (13.2) from the other one of the first and second patches (5).

5. The cardiac assist device (1) according to one of Claims 2 to 4, wherein the abutment element is a sleeve (10.1) and the pulling element a cable (13.1) running inside the sleeve (10.1).

6. The cardiac assist device (1) according to one of Claim 1 to 5, wherein the first patch (4) comprises a contact surface (7) for contacting the heart (6), optionally the left ventricle of the heart (6), and the second patch (5) a contact surface (8) for contacting the heart (6), optionally the right ventricle of the heart (6), the contact surface (7) of the first patch (4) being smaller in size than the contact surface (8) of the second patch (5).

7. The cardiac assist device (1) according to one of Claim 1 to 6, further comprising a fixation device (26; 27) for fixing at least one of the patches (4; 5) to the heart (6).

8. The cardiac assist device (1) according to Claim 7, wherein the fixation device (26; 27) comprises a suction port (28; 29) provided at the patch (4; 5) for fixing the patch (4; 5) to the heart (6) via vacuum.

9. The cardiac assist device (1) according to Claim 8, further comprising a vacuum source, optionally an ejector and/or a vacuum pump, which is optionally pneumatically driven, connected to the suction port (28; 29).

10. The cardiac assist device (1) according to one of Claims 7 to 9, wherein the fixation device (26; 27) comprises a needle (35) for piercing through the heart wall (6.1) and an unfoldable retaining element (36) at the needle's tip.

11. The cardiac assist device (1) according to Claim 10, the retaining element (36) being an umbrella-like structure (37) and/or an inflatable balloon (38).

12. The cardiac assist device (1) according to one of Claims 2 to 11, wherein the mechanical adjustment mechanism comprises an actuation device (16, 17), optionally a linear actuator, connected to the pulling element (13.1) and a control unit (20) for controlling the actuation device (16, 17).

13. The cardiac assist device (1) according to Claim 12, wherein the control unit (20) is configured to vary the positional relationship between the first and second patches (4, 5) via the actuation device (16, 17) periodically, optionally along a sinusoidal function.

14. The cardiac assist device (1) according to Claim 12 or 13, wherein the control unit (20) is configured to adjust the control of the actuation device (16, 17) based on a measured heart performance factor, optionally a p-V loop, to maximize heart performance.

15. The cardiac assist device (1) according to one of Claims 12 to 14, wherein the control unit (20) is configured to control the actuation device (16, 17) to synchronize the adjustment of the positional relationship between the first and the second patches (4, 5) with a measured contraction frequency of the heart (6).
